(19) Europäisches Patentamt
European Patent Office
Office européen des brevets

(11) EP 0 718 294 A1

(12) EUROPÄISCHE PATENTANMELDUNG

(43) Veröffentlichungstag: **26.06.1996 Patentblatt 1996/26**

(51) Int. Cl.[6]: **C07D 281/02**, C07D 283/02, C07D 285/36, A61K 31/55

(21) Anmeldenummer: **95118404.3**

(22) Anmeldetag: **23.11.1995**

(84) Benannte Vertragsstaaten:
**AT BE CH DE ES FR GB IT LI NL SE**

(30) Priorität: **16.12.1994 DE 4444930**

(71) Anmelder: **HOECHST AKTIENGESELLSCHAFT**
**D-65926 Frankfurt am Main (DE)**

(72) Erfinder:
- **Strobel, Hartmut, Dr.**
**D-63755 Alzenau (DE)**
- **Bohn, Helmut, Dr.**
**D-61137 Schöneck (DE)**
- **Klingler, Otmar, Dr.**
**D-63110 Rodgau (DE)**
- **Schindler, Ursula, Dr.**
**D-65812 Bad Soden (DE)**
- **Schönafinger, Karl, Dr.**
**D-63755 Alzenau (DE)**
- **Zoller, Gerhard, Dr.**
**D-61137 Schöneck (DE)**

# (54) 2-Amino-1,3-thiazepine und deren Verwendung als Hemmstoffe der Stickstoffmonoxid-Synthase

(57) Die vorliegende Erfindung betrifft 2-Amino-1,3-thiazepine der allgemeinen Formel I,

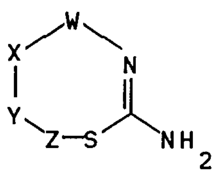

(I)

in der W für $CR^1R^2$, X für $CR^{20}R^{21}$, Y für $CR^{22}R^{23}$ und Z für $CR^{24}R^{25}$ steht und $R^1$, $R^2$, $R^{20}$, $R^{21}$, $R^{22}$, $R^{23}$, $R^{24}$ und $R^{25}$ die im Anspruch 1 angegebenen Bedeutungen haben, die Hemmstoffe der Stickstoffmonoxid-Synthase sind, und ihre Verwendung zur Behandlung von Krankheiten, die durch einen erhöhten Stickstoffmonoxid-Spiegel bedingt sind.

EP 0 718 294 A1

Printed by Rank Xerox (UK) Business Services
2.12.4/3.4

## Beschreibung

Die vorliegende Erfindung betrifft Thiazepine der allgemeinen Formel I,

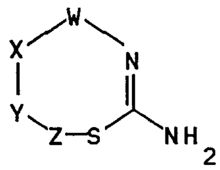

(I)

die aufgrund ihrer Fähigkeit zur Modulation der körpereigenen Stickstoffmonoxid-Produktion wertvolle Arzneimittel zur Vorbeugung und Bekämpfung von Krankheitszuständen sind, die durch einen gestörten Stickstoffmonoxid-Spiegel gekennzeichnet sind.

Stickstoffmonoxid (NO) spielt in verschiedensten physiologischen Prozessen eine bedeutende Rolle (siehe z.B. R.Henning, Nachr. Chem. Tech. Lab. 41 (1993), 413; J.F.Kerwin, M.Heller, Med. Res. Rev. 14 (1994), 23).

Es wirkt z.B. relaxierend auf die glatte Gefäßmuskulatur und ist auf diese Weise maßgeblich an der Regulation des Blutdrucks beteiligt, es steuert über eine Hemmung der Thrombocytenaggregation die Blutgerinnung, und es ist beispielsweise im Gehirn als Neurotransmitter in den Aufbau des Langzeitgedächtnisses involviert. In den NANC-Nerven des peripheren Nervensystems fungiert NO ebenfalls als Botenstoff. Die zelltoxische Wirkung des NO wird von Makrophagen für die Infektionsabwehr ausgenutzt.

Endogenes NO wird mit Hilfe mindestens drei verschiedener NO-Synthase-Isoenzyme aus Arginin gebildet.

Alle Isoenzyme benötigen NADPH, Flavinadenindinucleotid, Flavinmononucleotid und Tetrahydrobiopterin als Cofaktoren. Sie unterscheiden sich bezüglich ihrer Lokalisation im Organismus, ihrer Regulierbarkeit durch $Ca^{2+}$/Calmodulin sowie ihrer Induzierbarkeit durch Endotoxine und Cytokine. Die konstitutiven, calciumabhängigen NO-Synthasen finden sich beispielsweise im Endothel und im Gehirn und sind dort in die Regulation von Blutdruck und -gerinnung bzw. in Reizleitungsprozesse involviert. Die cytokin-induzierbare, calciumunabhängige Isoform tritt beispielsweise in Makrophagen, Glattmuskelzellen und Hepatocyten auf. Sie ist in der Lage, über einen langen Zeitraum relativ große Mengen NO zu produzieren und wird für entzündliche Prozesse und die zelltoxische Aktivität der Makrophagen verantwortlich gemacht.

Ein gestörter NO-Haushalt hat schwerwiegende Erkrankungen und Schädigungen zur Folge. So führt die exzessive Bildung von NO im septischen oder hämorrhagischen Schock zu massiven pathologischen Blutdruckabfällen. Übersteigerte NO-Produktion ist an der Entstehung von Typ-1-Diabetes und Atherosklerose beteiligt und scheint auch für die glutamatinduzierte Neurotoxizität nach cerebraler Ischämie verantwortlich zu sein. Hohe NO-Konzentrationen können darüberhinaus durch Desaminierung von Cytosin zu DNA-Schädigungen führen.

Der Ansatz, eine Modulation der NO-Produktion zur Behandlung dieser Krankheitsbilder zu verwenden, wurde bislang hauptsächlich mit Hilfe von Arginin-Analoga realisiert (GB-A-2 240 041; WO-A-93/13055; WO-A-93/24126; WO-A-94/02453; WO-A-94/07482). Als weitere potentielle NO-Synthase-Hemmstoffe werden in der Literatur N-Iminoethylornithin (McCall et al., Br.J.Pharmacol. 102 (1991), 234), Aminoguanidin (T.P.Misko et al., Eur.J.Pharmacol. 233 (1993), 119; EP-A-547588), Methylguanidin (US-A-5 246 971), Nitro- und Cyano-Aryle (WO-A-94/12163) sowie Pyrimidine, Pyridine, Pteridinone und Indazole (WO-A-94/14780) diskutiert. In der WO-A-94/12165 wird die hemmende Wirkung von Isothioharnstoffen auf die NO-Synthase offenbart. Neben den dort hauptsächlich untersuchten offenkettigen Isothioharnstoffen werden auch 2-Amino-1,3-thiazol-Derivate erwähnt.

Überraschend wurde nun gefunden, daß Thiazepin-Derivate der allgemeinen Formel I besonders gut geeignete Hemmstoffe der induzierbaren NO-Synthase sind und die NO-Bildung wesentlich stärker hemmen als die in der WO-A-94/12165 erwähnten Thiazol-Derivate. Sie sind somit zur Modulation der endogenen NO-Produktion geeignet und können als Wirkstoffe in Arzneimitteln zur Behandlung von Krankheiten verwendet werden, die durch einen überhöhten NO-Spiegel charakterisiert sind. Nur wenige Vertreter von Thiazepinen der allgemeinen Formel I wurden bisher in der Literatur beschrieben, die pharmakologischen Eigenschaften oder medizinische Verwendungen von Verbindungen der allgemeinen Formel I sind bisher aber nicht bekannt.

Gegenstand der vorliegenden Erfindung sind daher 2-Amino-1,3-thiazepine der allgemeinen Formel I,

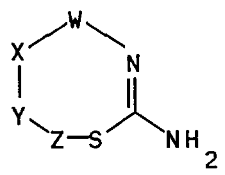

(I)

in der W für $CR^1R^2$ steht und
$R^1$ und $R^2$ unabhängig voneinander für Wasserstoff, Halogen, $R^3$, $R^4$, $OR^5$, $SR^{5'}$, $S(O)_p$-$R^6$, wobei p für 1 oder 2 steht, $NR^7R^8$, Cyan, Nitro, $COOR^9$, $CONR^{7'}R^{8'}$ oder $P(O)(OH)_2$ stehen; oder $R^1$ und $R^2$ zusammen für ($C_1$-$C_6$)-Alkyliden oder ($C_3$-$C_7$)-Cycloalkyliden, die auch jeweils einen oder mehrere Reste $R^{10}$ tragen können, oder $R^1$ und $R^2$ zusammen für =O, =S, =NH, =$NR^3$ oder =NOH stehen; oder $R^1$ und $R^2$ zusammen mit dem Kohlenstoffatom, an das sie gebunden sind, einen Rest der allgemeinen Formel II

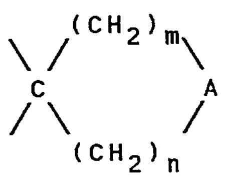

(II)

bilden, der vollständig oder teilweise dehydriert sein kann, der auch benzanelliert sein kann, der durch einen oder mehrere gleiche oder verschiedene Reste $R^{10}$ substituiert sein kann und worin m und n unabhängig voneinander für die Zahlen 0, 1, 2, 3, 4 oder 5 stehen, aber die Summe m + n einen der Werte 1, 2, 3, 4 oder 5 hat; oder $R^1$ oder $R^2$ eine freie Valenz bedeutet, die mit einer ebensolchen freien Valenz an einem Atom, das direkt an das $R^1$ und $R^2$ tragende Kohlenstoffatom gebunden ist, eine Doppelbindung bildet;
$R^3$ für ($C_1$-$C_{10}$)-Alkyl, ($C_2$-$C_{10}$)-Alkenyl, ($C_2$-$C_{10}$)-Alkinyl; ($C_3$-$C_7$)-Cycloalkyl, ($C_9$-$C_{11}$)-Benzocycloalkyl oder den Rest eines 4- bis 10-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, wobei der Alkyl-, Alkenyl-, Alkinyl-, Cycloalkyl- und der heterocyclische Rest durch einen oder mehrere gleiche oder verschiedene Reste $R^{10}$ substituiert sein können;
$R^4$ für Phenyl oder Naphthyl steht, die durch ein bis drei gleiche oder verschiedene Reste aus der Reihe ($C_1$-$C_4$)-Alkyl, ($C_1$-$C_4$)-Alkoxy, Halogen, Nitro, Trifluormethyl, Cyan, Hydroxy, ($C_1$-$C_4$)-Alkoxycarbonyl, Carboxyl, Carbamoyl, ($C_1$-$C_4$)-Alkyl-$S(O)_q$, wobei q für 0, 1 oder 2 steht, Amino, ($C_1$-$C_4$)-Alkylcarbonylamino, ($C_1$-$C_4$)-Alkylamino und Di(($C_1$-$C_4$)-Alkyl)amino substituiert sein können;
$R^5$ und $R^{5'}$ unabhängig voneinander für Wasserstoff, ($C_1$-$C_{10}$)-Alkyl, in dem die Alkylkette auch durch ein bis drei Sauerstoffatome unterbrochen sein kann, ($C_2$-$C_{10}$)-Alkenyl, ($C_3$-$C_7$)-Cycloalkyl, $R^4$, $R^4$-($C_1$-$C_6$)-Alkyl, den Rest eines 5- bis 7-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, ($C_1$-$C_{10}$)-Alkanoyl, $R^4$-CO, $R^4$-($C_1$-$C_6$)-Alkyl-CO, ($C_1$-$C_{10}$)-Alkyloxycarbonyl, $R^4$O-CO, $R^4$-($C_1$-$C_6$)-Alkoxy-CO oder einen Rest der allgemeinen Formel III

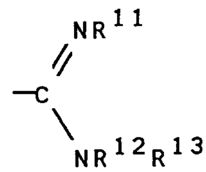

(III)

stehen;
$R^6$ für $(C_1\text{-}C_{10})$-Alkyl, $(C_2\text{-}C_{10})$-Alkenyl, $R^4$ oder $R^4$-$(C_1\text{-}C_6)$-Alkyl steht;
$R^7$ und $R^8$ unabhängig voneinander die für $R^5$ angegebenen Bedeutungen haben oder für $(C_1\text{-}C_6)$-Alkylsulfonyl oder $R^4$-$S(O)_2$ stehen oder $R^7$ und $R^8$ zusammen mit dem Stickstoffatom, an das sie gebunden sind, einen Rest der allgemeinen Formel IV

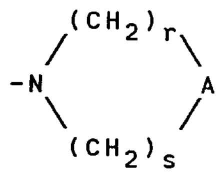

(IV)

bilden, der an den $CH_2$-Gruppen durch einen oder mehrere gleiche oder verschiedene Reste $R^{10}$ substituiert sein kann und worin r und s unabhängig voneinander für die Zahlen 1, 2, 3 oder 4 stehen, aber die Summe r + s kleiner als 6 ist;
$R^{7'}$ und $R^{8'}$ unabhängig von $R^7$ und $R^8$ die für $R^7$ und $R^8$ angegebenen Bedeutungen haben;
$R^9$ eine der Bedeutungen von $R^5$ hat, aber nicht für den Rest der allgemeinen Formel III steht;
$R^{10}$ für $(C_1\text{-}C_6)$-Alkyl, $(C_3\text{-}C_7)$-Cycloalkyl, den Rest eines 5- bis 7-gliedrigen Heterocyclus mit einem bis drei gleichen oder verschiedenen Heteroatomen aus der Reihe Stickstoff, Sauerstoff und Schwefel, der aromatisch, teilweise gesättigt oder vollständig gesättigt sein kann, $R^4$, Halogen, $OR^5$, $SR^{5'}$, $S(O)_p$-$R^6$, wobei p für 1 oder 2 steht, $NR^7R^8$, die Hydroximinogruppe, die Oxogruppe, Cyan, Nitro, $COOR^9$, $CONR^{7'}R^{8'}$ oder $P(O)(OH)_2$ steht;
die Reste $R^{11}$, $R^{12}$ und $R^{13}$ unabhängig voneinander für Wasserstoff, $(C_1\text{-}C_6)$-Alkyl, $(C_3\text{-}C_7)$-Cycloalkyl, $R^4$, $(C_1\text{-}C_6)$-Alkanoyl, $(C_1\text{-}C_6)$-Alkyl-$S(O)_2$ oder $R^4$-$S(O)_2$ stehen;
$R^{14}$ für $(C_1\text{-}C_4)$-Alkyl, Benzyl, Phenyl, $(C_1\text{-}C_6)$-Alkanoyl, $(C_1\text{-}C_6)$-Alkyl-$S(O)_2$ oder $R^4$-$S(O)_2$ steht;
A für eine Methylengruppe, die durch einen oder zwei gleiche oder verschiedene Reste $R^{10}$ substituiert sein kann, für Sauerstoff, Schwefel oder $NR^{14}$ steht;
X für $CR^{20}R^{21}$ steht und $R^{20}$ und $R^{21}$ unabhängig von den Bedeutungen der Reste $R^1$ und $R^2$ die für $R^1$ und $R^2$ angegebenen Bedeutungen haben;
Y für $CR^{22}R^{23}$ steht und $R^{22}$ und $R^{23}$ unabhängig von den Bedeutungen der Reste $R^1$ und $R^2$ die für $R^1$ und $R^2$ angegebenen Bedeutungen haben;
Z für $CR^{24}R^{25}$ steht und $R^{24}$ und $R^{25}$ unabhängig von den Bedeutungen der Reste $R^1$ und $R^2$ die für $R^1$ und $R^2$ angegebenen Bedeutungen haben;
wobei auch Reste der Gruppen W, X, Y und Z über eine Bindung verknüpft sein können, so daß ein Bicyclus oder Tricyclus gebildet wird;
und ihrer tautomeren Formen sowie ihrer pharmakologisch verträglichen Salze als pharmakologische Wirkstoffe.

Beispiele für Verbindungen der allgemeinen Formel I, in der ein Substituent in den Gruppen W, X, Y und Z eine freie Valenz bedeutet, die mit einer freien Valenz an einem direkt an diese Gruppe gebundenen Atom eine endocyclische Doppelbindung ausbildet, sind u.a. die Verbindungen der Formeln Va bis Vf.

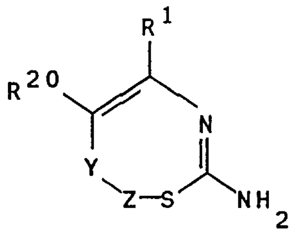 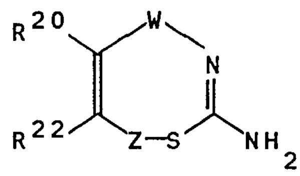 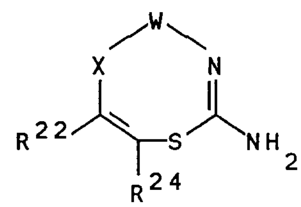

(Va) (Vb) (Vc)

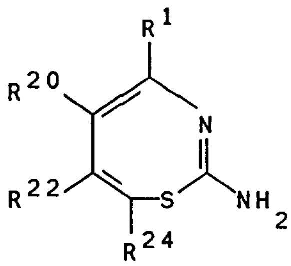 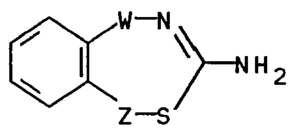 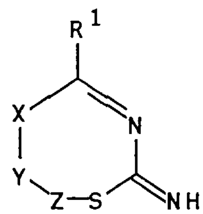

(Vd) (Ve) (Vf)

Halogen steht für Fluor, Chlor, Brom oder Iod, bevorzugt für Fluor oder Chlor.

Alkylgruppen können geradkettig oder verzweigt sein. Dies gilt auch, wenn sie in anderen Gruppen, beispielsweise in Alkoxy-, Alkylmercapto-, Alkyliden-, Alkoxycarbonyl- oder Alkanoylgruppen auftreten. Beispiele für Alkylgruppen, die in den erfindungsgemäß zu verwendenden Verbindungen der allgemeinen Formel als solche, also als ($C_1$-$C_4$)-, ($C_1$-$C_6$)- oder ($C_1$-$C_{10}$)-Alkyl, oder in anderen Gruppen auftreten können, sind Methyl, Ethyl, n-Propyl, i-Propyl-, n-Butyl, i-Butyl, sek-Butyl, tert-Butyl, n-Pentyl, 3-Methylbutyl, 2,2-Dimethylpropyl, n-Hexyl, n-Heptyl, n-Octyl, n-Nonyl oder n-Decyl. Bevorzugte Alkylgruppen sind Methyl, Ethyl, n-Propyl, i-Propyl, n-Butyl, i-Butyl und tert-Butyl.

Auch Alkenyl- und Alkinylreste können geradkettig oder verzweigt sein. Beispiele für Alkenylreste sind Vinyl, 1-Propenyl, Allyl, Butenyl, 3-Methyl-2-butenyl, für Alkinylreste Ethinyl, 1-Propinyl, Propargyl oder 5-Hexinyl.

Der ($C_3$-$C_7$)-Cycloalkylrest ist beispielsweise ein Cyclopropyl-, ein Cyclobutyl-, ein Cyclopentyl-, ein Cyclohexyl- oder ein Cycloheptylrest. Bevorzugte Cycloalkylreste sind der Cyclopentyl- und der Cyclohexylrest.

Der heterocyclische Rest mit 1, 2, 3 oder 4 Heteroatomen aus der Reihe Stickstoff, Sauerstoff und Schwefel kann aromatisch, teilweise gesättigt oder vollständig gesättigt sein und kann anelliert sein. Bevorzugte Ringgrößen des Heterocyclus sind 5-Ringe, 6-Ringe und 7-Ringe. Beispiele für Heterocyclen, von denen sich der Rest ableitet, sind Azetidin, Pyrrolidin, Pyrrol, Indol, Pyrazol, Imidazolidin, Imidazolin, Imidazol, 1,2,3-Triazol, 1,2,4-Triazol, Tetrazol, Tetrahydrofuran, Furan, 1,3-Dioxolan, Tetrahydrothiophen, Thiophen, Benzothiophen, 1,3-Dithiolan, 1,3-Oxazolin, 1,3-Oxazol, 1,3,4-Oxadiazol, Furazan, 1,3-Thiazolidin, 1,3-Thiazol, Piperidin, 1,2,5,6-Tetrahydropyridin, 1,4-Dihydropyridin, Pyridin, Chinolin, Isochinolin, Pyridazin, Pyrimidin, Piperazin, 1,2,3-Triazin, 1,3,5-Triazin, Perhydropyran, 1,3-Dioxan, 1,4-Dioxan, 1,3-Dithian, Dihydro-1,3-oxazin, Morpholin, Perhydro-1,4-thiazin, 5,6-Dihydro-4H-1,3-thiazin, Perhydroazepin, Pteridin und dessen Derivate. Die Reste können in beliebigen Positionen gebunden sein, Pyridylreste z.B. in der 2-, der 3- oder der 4-Position. Stickstoffheterocyclen können auch über ein Stickstoffatom gebunden sein.

Beispiele für Substituenten, die sich an Alkylresten und anderen Resten befinden können, sind Phenyl, Pyridyl, Methoxy, Hydroxy, Acetoxy, Acetyl, 2-Oxopropyl, Carboxy, Methoxycarbonyl, Ethoxycarbonyl, Methylmercapto, Methansulfonyl, Fluor, Aminocarbonyl, Diethylamino und Guanidino.

Beispiele für substituierte Alkylgruppen sind Benzyl, 2-Phenylethyl, Hydroxymethyl, Methoxycarbonylmethyl, Aminocarbonylmethyl und Diethylaminomethyl.

In monosubstituierten Phenylresten kann sich der Substituent in der 2-, der 3- oder der 4-Position befinden. Ist Phenyl zweifach substituiert, können die Substituenten in 2,3-, 2,4-, 2,5-, 2,6-, 3,4 oder 3,5-Position stehen. Naphthylreste können als 1-Naphthyl- und 2-Naphthylreste vorliegen und ebenfalls in beliebigen Positionen substituiert sein.

Beispiele für substituierte Derivate des als Arylrest bevorzugten Phenylrests sind 2-, 3- oder 4-Methylphenyl, 4-tert.-Butylphenyl, 2-, 3-oder 4-Methoxyphenyl, 3-Ethoxyphenyl, 2,3-, 3,4- oder 3,5-Dimethoxyphenyl, 3,4,5-Trimethoxyphenyl, 3-Aminophenyl, 3- oder 4-Dimethylaminophenyl, 4-Acetylaminophenyl, 2-, 3- oder 4-Fluorphenyl, 2,3- oder 3,4-Difluorphenyl, 2-, 3- oder 4-Chlorphenyl, 2,3-, 3,4-, 3,5- oder 2,6-Dichlorphenyl, 4-Bromphenyl, 4-Hydroxyphenyl, 3-Hydroxy-4-methoxyphenyl, 2-, 3- oder 4-Nitrophenyl, 3- oder 4-Trifluormethylphenyl, 2-, 3- oder 4-Cyanphenyl.

Für $R^5$ stehende Reste von Alkylketten, die durch Sauerstoffatome unterbrochen sind, sind beispielsweise 2-Methoxyethyl, 2-Ethoxyethyl, 2-(2-Methoxyethoxy)ethyl oder 2-(2-(2-Methoxyethoxy)ethoxy)ethyl.

Beispiele für Reste der allgemeinen Formel IV sind 1-Azetidinyl, Pyrrolidino, 2,5-Dimethylpyrrolidino, Piperidino, 2,6-Dimethylpiperidino, 2,2-Dimethylpiperidino, Morpholino, 1,4-Thiazin-4-yl, 3,3-Dimethyl-1,4-thiazin-4-yl, Piperazino, 4-Methylpiperazino, 4-Acetylpiperazino, 4-Methylsulfonylpiperazino, 4-Phenylpiperazino oder Azepino.

Die Verbindungen der allgemeinen Formel I können in verschiedenen tautomeren Formen und in verschiedenen stereoisomeren Formen vorliegen, so in der tautomeren Form der allgemeinen Formel VI.

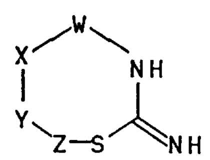

(VI)

Die vorliegende Erfindung umfaßt nicht nur alle tautomeren Formen, sondern auch alle stereoisomeren Formen, also z.B. reine Enantiomere, Enantiomerengemische und Racemate, reine Diastereomere und Diastereomerengemische oder cis/trans- bzw. E/Z-Isomere.

Geeignete Säuren für die Bildung pharmakologisch annehmbarer Säureadditionssalze der Verbindungen der allgemeinen Formel I sind beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden.

Verbindungen der allgemeinen Formel I, die eine saure Gruppe, beispielsweise eine Carbonsäuregruppe, enthalten, können an dieser mit anorganischen oder organischen Basen Salze bilden. Geeignete pharmakologisch annehmbare Salze sind beispielsweise Natriumsalze, Kaliumsalze, Magnesiumsalze, Calciumsalze, Ammoniumsalze oder Salze mit organischen Aminen, beispielsweise Ethanolamin oder Aminosäuren.

Bevorzugt ist das Thiazepin der allgemeinen Formel I, in der W, X, Y und Z gleichzeitig für die $CH_2$-Gruppe stehen.

Einige wenige Vertreter von Thiazepinen der allgemeinen Formel I sind als solche bekannt, siehe J. Lessel, Pharmazie 48 (1993), 812; L.I. Kirkovskii, Metalloorg. Khim. 3 (1990), 1115; T. Kato et al., Heterocycles 15 (1981), 399; J.B. Bream und J. Schmutz, Helv. Chim. Acta 60 (1977), 2872; JP-B-46/021713; DE-A-1965309; Y. Migalina et al., Ukr. Khim. Zh. 35 (1969), 526 und H.W. Schubert und O. Behner, Arch. Pharm. 301 (1968), 750.

Gegenstand der vorliegenden Erfindung sind auch die bisher nicht beschriebenen Verbindungen der allgemeinen Formel I als solche, also 2-Amino-1,3-thiazepine der allgemeinen Formel I,

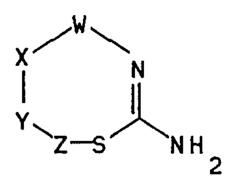

(I)

in der W für $CR^1R^2$ steht und

$R^1$ und $R^2$ unabhängig voneinander für Wasserstoff, Halogen, $R^3$, $R^4$, $OR^5$, $SR^{5'}$, $S(O)_p$-$R^6$, wobei p für 1 oder 2 steht, $NR^7R^8$, Cyan, Nitro, $COOR^9$, $CONR^{7'}R^{8'}$ oder $P(O)(OH)_2$ stehen; oder $R^1$ und $R^2$ zusammen für ($C_1$-$C_6$)-Alkyliden oder ($C_3$-$C_7$)-Cycloalkyliden, die auch jeweils einen oder mehrere Reste $R^{10}$ tragen können, oder $R^1$ und $R^2$ zusammen für

=O, =S, =NH, $=NR^3$ oder =NOH stehen; oder $R^1$ und $R^2$ zusammen mit dem Kohlenstoffatom, an das sie gebunden sind, einen Rest der allgemeinen Formel II

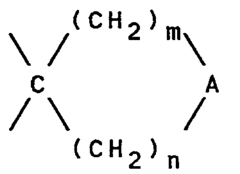

(II)

bilden, der vollständig oder teilweise dehydriert sein kann, der auch benzanelliert sein kann, der durch einen oder mehrere gleiche oder verschiedene Reste $R^{10}$ substituiert sein kann und worin m und n unabhängig voneinander für die Zahlen 0, 1, 2, 3, 4 oder 5 stehen, aber die Summe m + n einen der Werte 1, 2, 3, 4 oder 5 hat; oder $R^1$ oder $R^2$ eine freie Valenz bedeutet, die mit einer ebensolchen freien Valenz an einem Atom, das direkt an das $R^1$ und $R^2$ tragende Kohlenstoffatom gebunden ist, eine Doppelbindung bildet;

$R^3$ für $(C_1\text{-}C_{10})$-Alkyl, $(C_2\text{-}C_{10})$-Alkenyl, $(C_2\text{-}C_{10})$-Alkinyl; $(C_3\text{-}C_7)$-Cycloalkyl, $(C_9\text{-}C_{11})$-Benzocycloalkyl oder den Rest eines 4- bis 10-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, wobei der Alkyl-, Alkenyl-, Alkinyl-, Cycloalkyl- und der heterocyclische Rest durch einen oder mehrere gleiche oder verschiedene Reste $R^{10}$ substituiert sein können;

$R^4$ für Phenyl oder Naphthyl steht, die durch ein bis drei gleiche oder verschiedene Reste aus der Reihe $(C_1\text{-}C_4)$-Alkyl, $(C_1\text{-}C_4)$-Alkoxy, Halogen, Nitro, Trifluormethyl, Cyan, Hydroxy, $(C_1\text{-}C_4)$-Alkoxycarbonyl, Carboxyl, Carbamoyl, $(C_1\text{-}C_4)$-Alkyl-$S(O)_q$, wobei q für 0, 1 oder 2 steht, Amino, $(C_1\text{-}C_4)$-Alkylcarbonylamino, $(C_1\text{-}C_4)$-Alkylamino und Di($(C_1\text{-}C_4)$-Alkyl)amino substituiert sein können;

$R^5$ und $R^{5'}$ unabhängig voneinander für Wasserstoff, $(C_1\text{-}C_{10})$-Alkyl, in dem die Alkylkette auch durch ein bis drei Sauerstoffatome unterbrochen sein kann, $(C_2\text{-}C_{10})$-Alkenyl, $(C_3\text{-}C_7)$-Cycloalkyl, $R^4$, $R^4$-$(C_1\text{-}C_6)$-Alkyl, den Rest eines 5- bis 7-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, $(C_1\text{-}C_{10})$-Alkanoyl, $R^4$-CO, $R^4$-$(C_1\text{-}C_6)$-Alkyl-CO, $(C_1\text{-}C_{10})$-Alkyloxycarbonyl, $R^4$O-CO, $R^4$-$(C_1\text{-}C_6)$-Alkoxy-CO oder einen Rest der allgemeinen Formel III

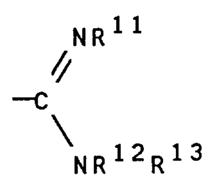

(III)

stehen;

$R^6$ für $(C_1\text{-}C_{10})$-Alkyl, $(C_2\text{-}C_{10})$-Alkenyl, $R^4$ oder $R^4$-$(C_1\text{-}C_6)$-Alkyl steht;

$R^7$ und $R^8$ unabhängig voneinander die für $R^5$ angegebenen Bedeutungen haben oder für $(C_1\text{-}C_6)$-Alkylsulfonyl oder $R^4$-$S(O)_2$ stehen oder $R^7$ und $R^8$ zusammen mit dem Stickstoffatom, an das sie gebunden sind, einen Rest der allgemeinen Formel IV

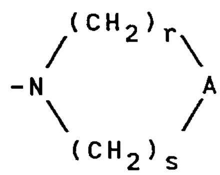

(IV)

bilden, der an den $CH_2$-Gruppen durch einen oder mehrere gleiche oder verschiedene Reste $R^{10}$ substituiert sein kann und worin r und s unabhängig voneinander für die Zahlen 1, 2, 3 oder 4 stehen, aber die Summe r + s kleiner als 6 ist;
$R^{7'}$ und $R^{8'}$ unabhängig von $R^7$ und $R^8$ die für $R^7$ und $R^8$ angegebenen Bedeutungen haben;
$R^9$ eine der Bedeutungen von $R^5$ hat, aber nicht für den Rest der allgemeinen Formel III steht;
$R^{10}$ für ($C_1$-$C_6$)-Alkyl, ($C_3$-$C_7$)-Cycloalkyl, den Rest eines 5- bis 7-gliedrigen Heterocyclus mit einem bis drei gleichen oder verschiedenen Heteroatomen aus der Reihe Stickstoff, Sauerstoff und Schwefel, der aromatisch, teilweise gesättigt oder vollständig gesättigt sein kann, $R^4$, Halogen, $OR^5$, $SR^{5'}$, $S(O)_p$-$R^6$, wobei p für 1 oder 2 steht, $NR^7R^8$, die Hydroximinogruppe, die Oxogruppe, Cyan, Nitro, $COOR^9$, $CONR^{7'}R^{8'}$ oder $P(O)(OH)_2$ steht;
die Reste $R^{11}$, $R^{12}$ und $R^{13}$ unabhängig voneinander für Wasserstoff, ($C_1$-$C_6$)-Alkyl, ($C_3$-$C_7$)-Cycloalkyl, $R^4$, ($C_1$-$C_6$)-Alkanoyl, ($C_1$-$C_6$)-Alkyl-$S(O)_2$ oder $R^4$-$S(O)_2$ stehen;
$R^{14}$ für ($C_1$-$C_4$)-Alkyl, Benzyl, Phenyl, ($C_1$-$C_6$)-Alkanoyl, ($C_1$-$C_6$)-Alkyl-$S(O)_2$ oder $R^4$-$S(O)_2$ steht;
A für eine Methylengruppe, die durch einen oder zwei gleiche oder verschiedene Reste $R^{10}$ substituiert sein kann, für Sauerstoff, Schwefel oder $NR^{14}$ steht;
X für $CR^{20}R^{21}$ steht und $R^{20}$ und $R^{21}$ unabhängig von den Bedeutungen der Reste $R^1$ und $R^2$ die für $R^1$ und $R^2$ angegebenen Bedeutungen haben;
Y für $CR^{22}R^{23}$ steht und $R^{22}$ und $R^{23}$ unabhängig von den Bedeutungen der Reste $R^1$ und $R^2$ die für $R^1$ und $R^2$ angegebenen Bedeutungen haben;
Z für $CR^{24}R^{25}$ steht und $R^{24}$ und $R^{25}$ unabhängig von den Bedeutungen der Reste $R^1$ und $R^2$ die für $R^1$ und $R^2$ angegebenen Bedeutungen haben;
wobei auch Reste der Gruppen W, X, Y und Z über eine Bindung verknüpft sein können, so daß ein Bicyclus oder Tricyclus gebildet wird;
und ihrer tautomeren Formen sowie ihrer pharmakologisch verträglichen Salze,
wobei aber,

a) wenn $R^{20}$ und $R^{21}$ für Wasserstoff stehen, nicht

a1) gleichzeitig $R^1$, $R^2$, $R^{22}$, $R^{23}$, $R^{24}$ und $R^{25}$ für Wasserstoff stehen können;
a2) gleichzeitig $R^{22}$, $R^{24}$ und $R^{25}$ für Wasserstoff, $R^{23}$ für die Carboxylgruppe und $R^1$ und $R^2$ zusammen für =O stehen können;
a3) gleichzeitig $R^{22}$, $R^{23}$ und $R^{24}$ für Wasserstoff, $R^{25}$ für Brommethyl und $R^1$ und $R^2$ zusammen für =O stehen können;
a4) gleichzeitig $R^1$, $R^2$, $R^{24}$ und $R^{25}$ für Wasserstoff und $R^{22}$ und $R^{23}$ zusammen für =O stehen können;
a5) gleichzeitig $R^1$ für Wasserstoff und $R^2$ für Butyl stehen und $R^{22}$, $R^{23}$, $R^{24}$ und $R^{25}$ zusammen mit den beiden Kohlenstoffatomen, an die sie gebunden sind, einen 2-Oxo-1,2-dihydrochinolin-3,4-diyl-Rest bilden können;

b) wenn $R^{20}$, $R^{21}$, $R^{22}$ und $R^{23}$ zusammen mit den beiden Kohlenstoffatomen, an die sie gebunden sind, einen 1,2-Phenylen-Rest oder einen 4-Chlor-1,2-phenylen-Rest bilden, nicht

b1) gleichzeitig $R^1$, $R^2$ und $R^{24}$ für Wasserstoff und $R^{25}$ für Phenyl stehen können;
b2) gleichzeitig $R^1$, $R^{24}$ und $R^{25}$ für Wasserstoff und $R^2$ für Phenyl stehen können;
b3) gleichzeitig $R^1$ und $R^2$ zusammen für =O und $R^{24}$ und $R^{25}$ zusammen für =O stehen können.

Hinsichtlich der Substituenten, wie Halogenatome, Alkylgruppen, etc. in den Thiazepinderivaten als solchen gilt das oben Gesagte entsprechend.

Die Synthese der Verbindungen der allgemeinen Formel I kann beispielsweise entsprechend dem folgenden Schema erfolgen:

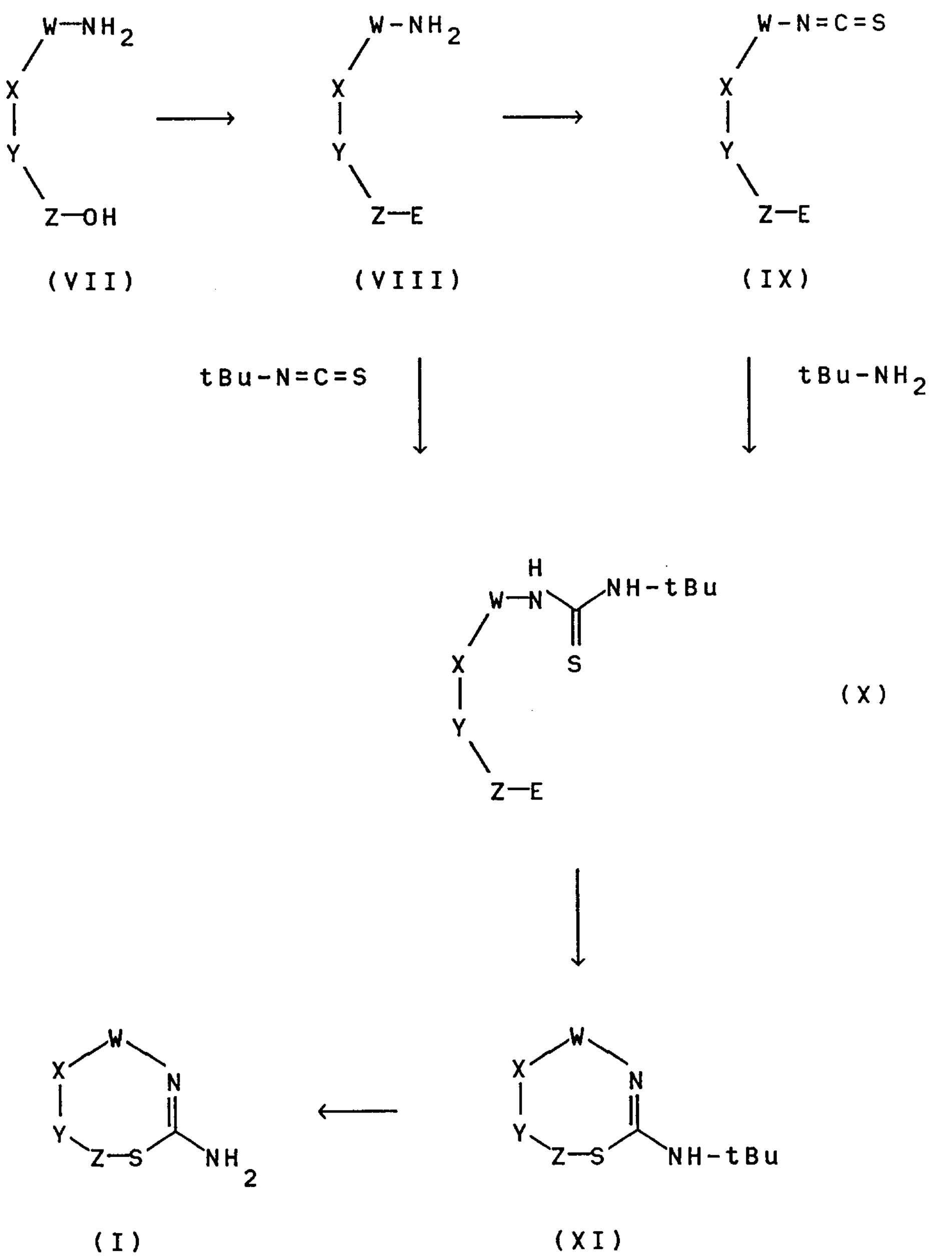


(VII) (VIII) (IX) (X) (I) (XI)

Ausgangsmaterial sind δ-Aminoalkohole der allgemeinen Formel VII, in der W, X, Y und Z die oben angegebenen Bedeutungen haben. Nach an sich bekannten Methoden werden daraus Verbindungen der allgemeinen Formel VIII hergestellt, in der E- eine Gruppe bedeutet, die in einer nucleophilen Substitutionsreaktion ausgetauscht werden kann. E- steht beispielsweise Cl-, Br-, I-, $CH_3SO_2O$-, $C_6H_5SO_2O$-, substituierte Benzolsulfonyloxy-Reste, z.B. Nitrobenzolsulfonyloxy oder Toluolsulfonyloxy, oder für eine andere aktivierte Form der Hydroxygruppe. Die Umwandlung der Hydroxygruppe in VII kann unter üblichen Bedingungen beispielsweise durchgeführt werden mit Thionylchlorid oder -bromid, mit Phosphorhalogeniden, wie Phosphorpentachlorid, Phosphortrichlorid oder Phosphortribromid, mit Brom- oder Iodwasserstoff oder mit aliphatischen oder aromatischen Sulfonsäurechloriden. Die Verbindungen der allgemeinen Formel VIII können mit tert-Butylisothiocyanat direkt in die Thioharnstoffe der allgemeinen Formel X überführt werden oder mit Reagenzien, wie Thiophosgen oder N,N'-Thiocarbonyldiimidazol zunächst in die Isothiocyanate der allgemeinen Formel IX umgewandelt werden, die mit tert-Butylamin die Thioharnstoffe der allgemeinen Formel X liefern, die zu den 2-tert-Butylamino-1,3-thiazepinen der allgemeinen Formel XI cyclisieren. Durch Abspaltung der tert-Butylgruppe, beispielsweise durch Kochen mit verdünnter Salzsäure, sind daraus die Verbindungen der allgemeinen Formel I erhältlich.

Ausgehend von den δ-Aminoalkoholen der allgemeinen Formel VII können auch zunächst mit tert-Butylisothiocyanat die Thioharnstoffe der allgemeinen Formel XII

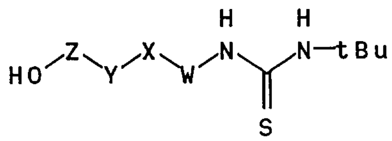

(XII)

hergestellt werden, die dann durch Umwandlung der Hydroxygruppe in die Gruppe E wie oben angegeben in die Verbindungen der allgemeinen Formel X überführt werden.

Ausgangsmaterial für einen anderen Syntheseweg sind die Verbindungen der allgemeinen Formel XIII,

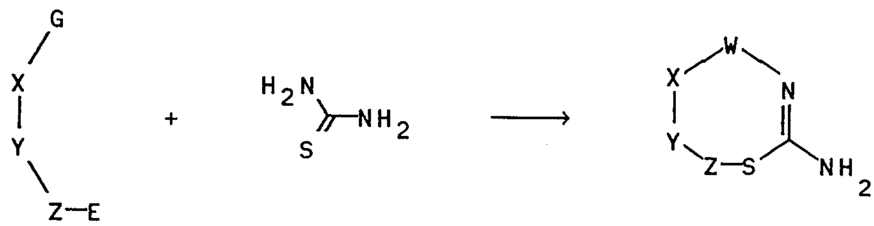

(XIII) (I)

in der G für eine Gruppe steht, die mit Aminogruppen in einer Substitutionsreaktion oder einer Additionsreaktion reagiert, beispielsweise die Carboxy-, Alkoxycarbonyl-, Aryloxycarbonyl-, Cyano- oder Formylgruppe, die Gruppe $-COR^1$ oder die Gruppe $-CR^1R^2-E'$, wobei $R^1$ und $R^2$ wie oben angegeben definiert sind, und in der E sowie E' für gleiche oder verschiedene Gruppen stehen, die - wie E in der allgemeinen Formel VIII - in einer nucleophilen Substitutionsreaktion ausgetauscht werden können, also z.B. für Chlor, Brom, Iod oder aliphatische oder aromatische Sulfonyloxy-Reste. Die Verbindungen der allgemeinen Formel XIII, die beipielsweise γ-Halogencarbonylverbindungen oder, wenn G- in der allgemeinen Formel XIII für -W-E' steht, α,δ-Dibromide, α,δ-Dichloride oder α,δ-Disulfonate sein können, liefern mit Thioharnstoff unter üblichen Bedingungen direkt die Thiazepine der allgemeinen Formel I.

Ein weiteres Syntheseverfahren ist die Umsetzung von Thioharnstoff mit Verbindungen der allgemeinen Formeln XIV,

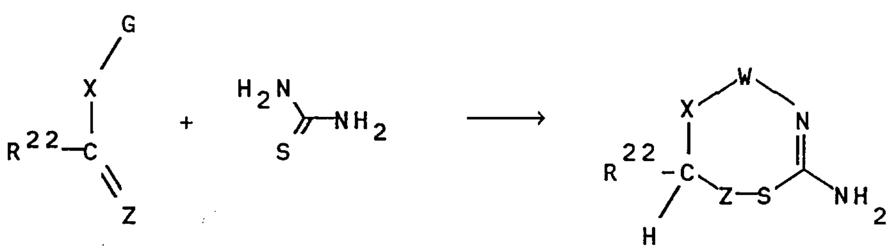

(XIV) (Ia)

in der die Doppelbindung zwischen der Gruppe Z und dem benachbarten Kohlenstoffatom durch elektronenziehende Substituenten aktiviert ist, so daß eine Addition des Schwefelatoms im Thioharnstoff an Z erfolgen kann, und in der G die für die Formel XIII angegebene Bedeutung hat.

Die Angaben zur Synthese von Verbindungen der allgemeinen Formel I in J.B. Bream und J. Schmutz, Helv. Chim. Acta 60 (1977), 2872; JP-B-46/021713 und H.W. Schubert und O. Behner, Arch. Pharm. 301 (1968), 750 sind in vollem Umfang Bestandteil der vorliegenden Offenbarung.

Die erfindungsgemäße Hemmung der NO-Freisetzung durch die Verbindungen der allgemeinen Formel I kann durch einen Aktivitätsassay bestimmt werden, der auf Arbeiten von Bredt und Snyder sowie Schmidt et al. basiert (s. D.S. Bredt und S.S. Snyder, Isolation of nitric oxide synthase, a calmodulin-requiring enzyme, Proc. Natl. Acad. Sci. USA 87 (1990), 682; H.H.H.W.Schmidt et al., Purification of a soluble isoform of guanylyl cyclase-activating factor synthase, Proc. Natl. Acad. Sci. USA 88 (1991), 365). Hierbei wird für gereinigte NO-Synthase (NOS) das bei der NO-Bildung anfallende Coprodukt L-Citrullin quantitativ erfaßt. Dies geschieht durch den Einsatz von $^{3}$H-radiomarkiertem L-Arginin als Substrat der Enzymreaktion, das zu $^{3}$H-L-Citrullin und NO umgesetzt wird. Nach Beendigung der Enzyminkubation wird entstandenes L-Citrullin von unverbrauchtem L-Arginin mittels Ionenaustauschchromatographie aus dem Reaktionsgemisch entfernt; die durch Flüssigkeitsszintillationsmessung ermittelte $^{3}$H-Aktivität entspricht dann der Menge an L-Citrullin.

Die Hemmung der NO-Freisetzung durch die Verbindungen der allgemeinen Formel I kann auch durch NO-Messung mittels Oxyhämoglobin auf Makrophagen wie folgt bestimmt werden:
Mäusemakrophagen (MM) des Stammes RAW 264.7 werden in Petrischalen (10 cm Durchmesser) angezüchtet. Als Nährmedium wird DMEM (Fa. Sigma Nr. D 5405), konditioniert mit 4 mM I-Glutamin, 3,5 g D-Glukose/l, 50 U/50 µg/ml Pen/Strep und 10 % FKS, eingesetzt unter 5 % $CO_2$, 95 % Luftfeuchtigkeit, 37°C. Die subkonfluenten Zellen werden mit dem Zellschaber abgekratzt, in Medium aufgenommen, die Zellzahl bestimmt (Zählung in Trypanblaulösung 0,2 %) und in 24-Napfplatten (1 x $10^{6}$ Zellen/ml, 1 ml/Napf) ausgesät.

Ca. 18 Stunden nach Aussaat werden die gut adherierten Mausmakrophagen mit LPS (Lipopolysaccharid von E. coli Serotyp 0111:B4, Fa. Sigma Nr. L 2630) und γ-Interferon (γIFN, Maus rekombinant, Firma Boehringer Mannheim Nr. 1276905) stimuliert. Dazu wird das Kulturmedium von den Zellen abgesaugt und 1 ml/Napf (24-Napfplatte) Inkubationsmedium (MEM ohne Phenolrot (Biochrom F0385)), konditioniert mit 4mM Glutamin, 3,5 g Glukose/l, 3,7 g $NaHCO_3$/l, 110 mg Natriumpyruvat/l, 50 U/50 µg/ml Pen/Strep und 5 % FKS, aufgegeben. Pro ml Inkubationsmedium werden 1 µl LPS und 1 µl γIFN pipettiert.

Daraus ergeben sich folgende Konzentrationen der Stimulatoren:

140 ng LPS/ml + 5 U γIFN/ml

Während der Stimulation über 4 Stunden wird die Neusynthese des NO-produzierenden Enzyms NO-Synthase (iNOS) induziert. Nach diesem Inkubationszeitraum wird das Inkubationsmedium abgesaugt und jeder Napf zweimal mit frischem Inkubationsmedium gespült. Versuchsmedium (bestehend aus dem Inkubationsmedium mit Zusatz von SOD (30 U/ml), Katalase (290 U/ml), Indomethacin (3,5 µg/ml), OxyHb (ca. 8 µM) und Testsubstanz (0,05-250 µM)) wird in die Näpfe gegeben (1 ml/Napf bei 24-Napfplatte). Als Kontrolle wird das konditionierte Versuchsmedium nur mit dem Lösungsmittel der Testsubstanz versetzt. Die mit Versuchsmedium beschickten Platten werden 120 Minuten im Brutschrank bei 37°C, 5 % $CO_2$ und 95 % Luftfeuchtigkeit inkubiert. Nach der 120-minütigen Inkubation wird das Versuchsmedium in Kunststoff-Halbmikroküvetten pipettiert und die Extinktionsdifferenz 576-592 nm am DU 70 gemessen.

Je 2 Näpfe werden mit einer Testsubstanz beschickt (n = 2), auf jeder Platte wird eine Kontrolle unstimuliert, eine Stimulationskontrolle und eine Positiv-Kontrolle (Nitro-L-Arginin, 500 µM) vergleichend zu den Testsubstanzen untersucht. Als Maßangabe für die Testsubstanzen wird die Extinktionsdifferenz 576-592 nm prozentual zur Stimulationskontrolle verglichen. Von dem mit Testsubstanz versetzten Versuchsmedium wird zum Zeitpunkt 0 die Extinktionsdifferenz 576-592 nm gemessen sowie der pH-Wert und die Osmolalität bestimmt.

Nach dem Abpipettieren des Versuchsmediums wird die Cytotoxizität der Testsubstanz geprüft (Zellviabilität).

Krankheiten, die durch einen erhöhten NO-Spiegel entstehen und die somit erfindungsgemäß mit den Verbindungen der allgemeinen Formel I behandelt werden können bzw. denen mit diesen vorgebeugt werden kann, sind insbesondere pathologische Blutdruckabfälle, wie sie beim septischen oder hämorrhagischen Schock, bei der Tumor- bzw. Krebstherapie mit Cytokinen oder bei der Leberzirrhose auftreten. Des weiteren entzündliche Erkrankungen, wie rheumatoide Arthritis und insbesondere Colitis ulcerosa, sowie Insulin-abhängiger Diabetes mellitus und Transplantat-Abstoßungsreaktionen.

Aber auch die folgenden Erkrankungen stehen im Zusammenhang mit einer gesteigerten Produktion von Stickstoffmonoxid und können erfindungsgemäß behandelt bzw. vorgebeugt werden. Im Bereich Herz-Kreislauf sind dies Arteriosklerose, postischämische Gewebeschäden und Infarktschäden, Reperfusionsschäden, Myokarditis auf der Grundlage einer Coxsackie-Virus-Infektion und Kardiomyopathie; im Bereich Nervensystem/Zentrales Nervensystem Neuritiden unterschiedlicher Ätiogenese (Neuritisformen), Enzephalomyelitiden, virale neurodegenerative Erkrankungen, Morbus Alzheimer, Hyperalgesie, Epilepsie, Migräne sowie Opioid-Entzugssymptome; im Bereich Niere akutes Nierenversagen sowie Nephritiden unterschiedlicher Ätiogenese, speziell Glomerulonephritis.

Außerdem sind auch Behandlungen im Bereich des Magens und des Uterus/der Plazenta sowie eine Beeinflussung der Motilität der Spermien Anwendungsgebiete für die Verbindungen der allgemeinen Formel I.

Die Verbindungen der allgemeinen Formel I und ihre pharmakologisch annehmbaren Salze können als Hilfsstoffe in biochemischen und pharmakologischen Untersuchungen in der Forschung und in Diagnoseverfahren eingesetzt werden, und sie können am Tier, bevorzugt am Säugetier, und insbesondere am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Salzes davon, neben üblichen pharmazeutisch einwandfreien Träger- und Zusatzstoffen, enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen oder Infusionslösungen, oder perkutan, z.B. in Form von Salben oder Tinkturen, erfolgen.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer pharmakologisch annehmbaren Salze und noch andere therapeutisch wirksame Stoffe enthalten.

Derartige andere therapeutisch wirksame Substanzen sind beispielsweise: β-Rezeptorenblocker, wie z.B. Propranolol, Pindolol, Metoprolol; Vasodilatatoren, wie z.B. Carbocromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon; entzündungshemmende Substanzen, wie etwa Corticosteroide, Salicylate oder Propionsäurederivate, wie beispielsweise Ibuprofen; Antibiotika, wie z.B. Penicilline oder Cephalosporine; NO-Donoren, wie z.B. organische Nitrate oder Sydnonimine oder Furoxane.

Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, in ähnlichen Mengenbereichen, d.h. im allgemeinen ebenfalls bei 0,5 bis 100 mg/Mensch. Die Tagesdosis kann in mehrere, z.B. 2 bis 4, Teilverabreichungen aufgeteilt werden.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Beispiel 1

2-Amino-4,5,6,7-tetrahydro-1,3-thiazepin-Hydrochlorid

a) 2-Amino-4,5,6,7-tetrahydro-1,3-thiazepin-Hydrochlorid wurde entsprechend H.W. Schubert und O. Behner, Arch. Pharm. 301 (1968), 750 hergestellt. Die physikalischen Daten entsprachen den dortigen Angaben. 1H-NMR (DMSO, 300 MHz) : 1,67 ppm (quintett, 2H, H-6); 1,94 (quintett, 2H, H-5); 3,09 - 3,18 (m, 2H, H-7); 3,24 (q, 2H, H-4); 9,20 (s, 1H, NH); 9,53 (s, 1H, NH); 10,34 (s, 1H, NH).

b) Die pharmakologische Wirksamkeit wurde wie oben beschrieben durch NO-Messung mittels Oxyhämoglobin auf Makrophagen bestimmt. Der $IC_{50}$-Wert betrug $1{,}3 \cdot 10^{-6}$ M.

Beispiel 2

3-Amino-1,5-dihydrobenzo[e][1,3]thiazepin-Hydrochlorid

a) 2-Aminomethylbenzylalkohol-Hydrochlorid

20 g (0,13 mol) 2-Hydroxymethylbenzoesäureamid in 450 ml Tetrahydrofuran werden zu einer siedenden Suspension von Lithiumaluminiumhydrid in 500 ml Tetrahydrofuran getropft. Das Gemisch wird 5 h unter Rückfluß erhitzt, über Nacht bei Raumtemperatur gerührt und tropfenweise mit 91 ml gesättigter Kaliumcarbonatlösung versetzt. Der Niederschlag wird abgesaugt und mit Tetrahydrofuran nachgewaschen. Die vereinigten Filtrate werden eingeengt. Der Rückstand wird in 100 ml Isopropanol aufgenommen, mit etherischer Salzsäure versetzt, der sich bildende Niederschlag abgesaugt und aus Isopropanol umkristallisiert.
Ausbeute: 19,2 g. Schmp. 225° C

b) 3-Amino-1,5-dihydrobenzo[e][1,3]thiazepin-Hydrochlorid

7 g (40 mol) 2-Aminomethylbenzylalkohol-Hydrochlorid werden in 80 m Ethanol suspendiert, eine Lösung von Kaliumethylat in 10 ml Ethanol wird zugegeben und das Gemisch wird 10 min auf 50° C erwärmt. Anschießend wird auf 5° C abgekühlt und vom ausgefallenen Kaliumchlorid abgesaugt. Zum Filtrat werden 4,64 g (40 mol) tert-Butylisothiocyanat getropft und das Reaktionsgemisch wird 4 h unter Rückfluß erhitzt. Nach Rühren über Nacht wird am Rotationsverdampfer eingeengt, der Rückstand in 30 ml wäßriger Bromwasserstoffsäure (48 %ig) aufgenommen und 2 h unter Rückfluß erhitzt. Das auf diese Weise erhaltene Reaktionsgemisch wird filtriert, am Rotationsverdampfer eingeengt und an Kieselgel mit Dichlormethan/Methanol/Wasser/Essigsäure (85/15/2/2) chromatographiert. Die Hauptfraktion wird aus Ethanol umkristallisiert.
Ausbeute: 0,88 g weiße Kristalle. Schmp. 246° C (Zers.)

Beispiel 3

2-Amino-5-phenyl-4,5,6,7-tetrahydro-1,3-thiazepin-Hydrochlorid

a) 4-Amino-3-phenylbutanol-Hydrochlorid

12,7 g (0,33 mol) Lithiumaluminiumhydrid werden in 250 ml Diethylether vorgelegt, es wird auf 0° C gekühlt und 25 g (0,11 mol) 4-Nitro-3-phenylbuttersäuremethylester (M.J. Leonard et al., J. Am. Chem. Soc. 73 (1951), 857), gelöst in 100 ml Diethylether, werden zugetropft. Anschließend wird 2 h unter Rückfluß erhitzt, mit gesättigter Kaliumcarbonatlösung hydrolysiert, mit Wasser versetzt und 1 h bei Raumtemperatur gerührt. Der Niederschlag wird abgesaugt und mit Dichlormethan ausgekocht. Die vereinigten organischen Phasen werden eingeengt. Der Rückstand wird in 1 N Salzsäure aufgenommen und mit Diethylether extrahiert. Die wäßrige Phase wird mit Natronlauge basisch gestellt und mit Dichlormethan extrahiert. Der nach Einengen verbeibende Rückstand wird in Tetrahydrofuran aufgenommen und durch Zugabe von etherischer Salzsäure das Hydrochlorid gefällt. Man erhält 10,4 g des Hydrochlorids, die ohne weitere Reinigung in die Folgereaktion eingesetzt werden.

b) 4-Chlor-2-phenylbutylamin-Hydrochlorid

Zu einer Suspension von 14,1 g (68 mmol) Phosphorpentachlorid und 5,2 g (52 mmol) Caliumcarbonat in 100 ml Chloroform wird bei 0° C eine Lösung von 10,4 g (52 mmol) 4-Amino-3-phenylbutanol-Hydrochlorid in 50 ml Chloroform zugetropft. Anschließend wird vom Salz abfiltriert, eingeengt und der verbleibende Rückstand ohne weitere Reinigung in die Folgereaktion eingesetzt.

c) 4-Chlor-2-phenylbutylisothiocyanat

11.4 g (52 mmol) 4-Chlor-2-phenylbutylamin-Hydrochlorid werden nacheinander mit 50 ml Wasser, 50 ml Ethylenchorid und 10,4 g (104 mmol) Calciumcarbonat versetzt. Unter Kühlung werden bei 10° C 6,9 g (60 mmol) Thiophosgen in 10 ml Ethylenchlorid zugetropft. Es wird 16 h bei Raumtemperatur gerührt und schließlich vom Salz abgesaugt. Die Phasen des Filtrats werden getrennt. Nach Trocknen der organischen Phase und Abziehen des Lösungsmittels wird im Hochvakuum destilliert.
Ausbeute: 3,2 g schwach gelbes Öl. Sdp. (0,012 mbar) 130° C.

d) 2-Amino-5-phenyl-4,5,6,7-tetrahydro-1,3-thiazepin-Hydrochlorid

3,1 g (14 mmol) 4-Chlor-2-phenylbutylisothiocyanat, gelöst in 5 ml Xylol, werden zu einer Lösung von 2,0 g (28 mmol) tert-Butylamin in 15 ml Xylol getropft und 2 h unter Rückfluß erhitzt. Anschießend wird vom ausgefallenen Salz abfiltriert und dreimal mit jeweils 10 ml 2 N Salzsäure extrahiert. Die vereinigten wäßrigen Phasen werden bis zur vollständigen Umsetzung unter Rückfluß erhitzt. Zur Aufarbeitung wird am Rotationsverdampfer eingeengt und nach Chromatographie an Kieselgel mit Butanol/Essigsäure/Wasser (8/2/2) (org. Phase) aus Ethanol/Essigsäureethylester/Hexan kristallisiert.
Ausbeute: 0,5 g weiße Kristalle. Schmp. 234 - 235 ° C.

Beispiel 4

2-Amino-6-phenyl-4,5,6,7-tetrahydro-1,3-thiazepin-Hydrochlorid

a) 4-Nitro-2-phenylbuttersäureethylester

Zu 179,8 g (2,95 mo) Nitromethan in 200 ml tert-Butanol werden 147,7 g (0,34 mol) Triton B (methanolische Lösung) zugetropft und es wird 30 min bei Raumtemperatur gerührt. Zu dieser Mischung werden 86,5 g (0,49 mol) 2-Phenylacrylsäureethylester (Chem. Ber. 119 (1986), 3694), gelöst in 30 m tert-Butanol, zugetropft und es wird 4 h bei 70° C gerührt. Zur Aufarbeitung wird am Rotationsverdampfer eingeengt, in Dichlormethan aufgenommen, mit Wasser gewaschen und erneut eingeengt. Der Rückstand wird mit Hexan/Essigsäureethylester (2/1) über Kieselgel filtriert und schließich im Hochvakuum fraktioniert destilliert. Man erhält 26,7 g farbloses Öl. Sdp. (0,5 mm) 148° C.

b) 4-Amino-2-phenylbutanol-Hydrochlorid

Entsprechend der Herstellung von 4-Amino-3-phenylbutanol-Hydrochlorid (Beispiel 3a) werden aus 92,6 g (0,39 mol) 4-Nitro-2-phenylbuttersäureethylester und 47,2 g (1,24 mol) Lithiumaluminiumhydrid 42 g Produkt erhalten.

c) 4-Chlor-3-phenylbutylamin-Hydrochlorid

Entsprechend der Herstellung von 4-Chlor-2-phenylbutylamin-Hydrochlorid (Beispiel 3b) werden aus 19,3 g (96 mmol) 4-Amino-2-phenylbutanol-Hydrochlorid 22,2 g Rohprodukt erhalten, die ohne weitere Reinigung in die Folgereaktion eingesetzt werden.

d) 4-Chlor-3-phenylbutylisothiocyanat

Entsprechend der Herstellung von 4-Chlor-2-phenylbutylisothiocyanat (Beispiel 3c) werden aus 22,2 g (0,1 mol) 4-Chlor-3-phenylbutylamin-Hydrochlorid 3,94 g Produkt erhalten. Sdp. (0,04 mbar) 140° C.

e) 2-Amino-6-phenyl-4,5,6,7-tetrahydro-1,3-thiazepin-Hydrochlorid

Zu einer Lösung von 2,64 g (36mmol) tert-Butylamin in 20 ml Xylol werden 3,7 g (16 mmol) 4-Chlor-3-phenylbutylisothiocyanat zugetropft und 8 h unter Rückfluß erhitzt. Nach 16 h Rühren bei Raumtemperatur wird abgesaugt, das Fitrat wird dreimal mit 2 N Salzsäure extrahiert und die vereinigten Extrakte werden 2 h unter Rückfluß erhitzt. Das Reaktionsgemisch wird am Rotationsverdampfer eingeengt und an Kieselgel mit Dichlormethan/Methanol/Essigsäure (50/10/1) chromatographiert. Die Hauptfraktion wird eingeengt, in 2 N Salzsäure aufgenommen und gefriergetrocknet. Das verbleibende Öl kristallisiert beim Vereiben mit Diethylether durch.
Ausbeute: 0,33 g beige Kristalle. Schmp. 127 ° C (Zers.)

Beispiel 5

2-Amino-4,7-dihydro-1,3-thiazepin-Hydrochlorid

a) 4-Chlorbut-2-enylisothiocyanat

Entsprechend der Herstellung von 4-Chlor-2-phenylbutylisothiocyanat (Beispiel 3c) werden aus 30 g (0,21 mol) 4-Chlorbut-2-enylamin-Hydrochlorid (Synthesis (1988), 347), 27,9 g (0,24 mol) Thiophosgen und 42,3 g (0,42 mol) Calciumcarbonat 22,1 g Produkt in Form eines farblosen Öls erhalten. Sdp. (2 mm) 80° C.

b) 2-Amino-4,7-dihydro-1,3-thiazepin-Hydrochlorid Entsprechend der Herstellung von 2-Amino-5-phenyl-4,5,-6,7-tetrahydro-1,3-thiazepin-Hydrochlorid (Beispiel 3d) werden aus 20,7 g (0,14 mol) 4-Chlorbut-2-enylisothiocyanat und 20,5 g (0,28 mol) tert-Butylamin nach Abspaltung des tert-Butylrestes mit halbkonz. Salzsäure 3,3 g Produkt erhalten.
Beige Kristalle. Schmp. 123 - 125° C.

Die nachfolgenden Beispiele betreffen pharmazeutische Zubereitungsformen.

**Beispiel A**

Gelatineweichkapseln, enthaltend 100 mg Wirkstoff pro Kapsel:

| | **pro Kapsel** |
|---|---|
| Wirkstoff | 100 mg |
| aus Kokosfett fraktioniertes Triclycerid-Gemisch | 400 mg |
| Kapselinhalt | 500 mg |

**Beispiel B**

Injektionslösung, enthaltend 2,0 mg Wirkstoff pro ml:

| | **pro ml** |
|---|---|
| Wirkstoff | 2,0 mg |
| Polyethylenglycol 400 | 5,0 mg |
| Natriumchlorid | 2,7 mg |
| Wasser zu Injektionszwecken | ad 1 ml |

**Beispiel C**

Emulsion, enthaltend 60 mg Wirkstoff pro 5 ml:

| | **pro 100 ml Emulsion** |
|---|---|
| Wirkstoff | 1,2 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

**Beispiel D**

Rektale Arzneiform, enthaltend 40 mg Wirkstoff pro Suppositorium:

| | **pro Suppositorium** |
|---|---|
| Wirkstoff | 40 mg |
| Suppositoriengrundmasse | ad 2 g |

**Beispiel E**

Tabletten, enthaltend 40 mg Wirkstoff pro Tablette:

| | **pro Tablette** |
|---|---|
| Wirkstoff | 40 mg |
| Lactose | 600 mg |
| Maisstärke | 300 mg |
| lösliche Stärke | 20 mg |
| Magnesiumstearat | 40 mg |
| | 1000 mg |

**Beispiel F**

Dragees, enthaltend 50 mg Wirkstoff pro Dragee:

| | **pro Dragee** |
|---|---|
| Wirkstoff | 50 mg |
| Maisstärke | 100 mg |
| Lactose | 60 mg |
| sec. Calciumphosphat | 30 mg |
| lösliche Stärke | 5 mg |
| Magnesiumstearat | 10 mg |
| kolloidale Kieselsäure | 5 mg |
| | 260 mg |

**Beispiel G**

Für die Herstellung des Inhalts von Hartgelatinekapseln eignen sich die folgenden Rezepturen:

| | | |
|---|---|---|
| a) | Wirkstoff | 100 mg |
| | Maisstärke | 300 mg |
| | | 400 mg |
| b) | Wirkstoff | 140 mg |
| | Milchzucker | 180 mg |
| | Maisstärke | 180 mg |
| | | 500 mg |

**Beispiel H**

Tropfen können nach folgender Rezeptur hergestellt werden (100 mg Wirkstoff in 1 ml = 20 Tropfen):

| | |
|---|---|
| Wirkstoff | 10 g |
| Benzoesäuremethylester | 0,07 g |
| Benzoesäureethylester | 0,03 g |
| Ethanol 96 %ig | 5 ml |
| entmineralisiertes Wasser | ad 100 ml |

**Patentansprüche**

**1.** 2-Amino-1,3-thiazepine der allgemeinen Formel I,

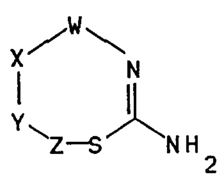

(I)

in der W für $CR^1R^2$ steht und
$R^1$ und $R^2$ unabhängig voneinander für Wasserstoff, Halogen, $R^3$, $R^4$, $OR^5$, $SR^{5'}$, $S(O)_p$-$R^6$, wobei p für 1 oder 2 steht, $NR^7R^8$, Cyan, Nitro, $COOR^9$, $CONR^{7'}R^{8'}$ oder $P(O)(OH)_2$ stehen; oder $R^1$ und $R^2$ zusammen für ($C_1$-$C_6$)-Alkyliden oder ($C_3$-$C_7$)-Cycloalkyliden, die auch jeweils einen oder mehrere Reste $R^{10}$ tragen können, oder $R^1$ und $R^2$ zusammen für =O, =S, =NH, =$NR^3$ oder =NOH stehen; oder $R^1$ und $R^2$ zusammen mit dem Kohlenstoffatom, an das sie gebunden sind, einen Rest der allgemeinen Formel II

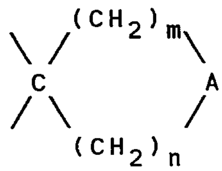 (II)

bilden, der vollständig oder teilweise dehydriert sein kann, der auch benzanelliert sein kann, der durch einen oder mehrere gleiche oder verschiedene Reste $R^{10}$ substituiert sein kann und worin m und n unabhängig voneinander für die Zahlen 0, 1, 2, 3, 4 oder 5 stehen, aber die Summe m + n einen der Werte 1, 2, 3, 4 oder 5 hat; oder $R^1$ oder $R^2$ eine freie Valenz bedeutet, die mit einer ebensolchen freien Valenz an einem Atom, das direkt an das $R^1$ und $R^2$ tragende Kohlenstoffatom gebunden ist, eine Doppelbindung bildet;

$R^3$ für ($C_1$-$C_{10}$)-Alkyl, ($C_2$-$C_{10}$)-Alkenyl, ($C_2$-$C_{10}$)-Alkinyl; ($C_3$-$C_7$)-Cycloalkyl, ($C_9$-$C_{11}$)-Benzocycloalkyl oder den Rest eines 4- bis 10-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, wobei der Alkyl-, Alkenyl-, Alkinyl-, Cycloalkyl- und der heterocyclische Rest durch einen oder mehrere gleiche oder verschiedene Reste $R^{10}$ substituiert sein können;

$R^4$ für Phenyl oder Naphthyl steht, die durch ein bis drei gleiche oder verschiedene Reste aus der Reihe ($C_1$-$C_4$)-Alkyl, ($C_1$-$C_4$)-Alkoxy, Halogen, Nitro, Trifluormethyl, Cyan, Hydroxy, ($C_1$-$C_4$)-Alkoxycarbonyl, Carboxyl, Carbamoyl, ($C_1$-$C_4$)-Alkyl-S(O)$_q$, wobei q für 0, 1 oder 2 steht, Amino, ($C_1$-$C_4$)-Alkylcarbonylamino, ($C_1$-$C_4$)-Alkylamino und Di(($C_1$-$C_4$)-Alkyl)amino substituiert sein können;

$R^5$ und $R^{5'}$ unabhängig voneinander für Wasserstoff, ($C_1$-$C_{10}$)-Alkyl, in dem die Alkylkette auch durch ein bis drei Sauerstoffatome unterbrochen sein kann, ($C_2$-$C_{10}$)-Alkenyl, ($C_3$-$C_7$)-Cycloalkyl, $R^4$, $R^4$-($C_1$-$C_6$)-Alkyl, den Rest eines 5- bis 7-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, ($C_1$-$C_{10}$)-Alkanoyl, $R^4$-CO, $R^4$-($C_1$-$C_6$)-Alkyl-CO, ($C_1$-$C_{10}$)-Alkyloxycarbonyl, $R^4$O-CO, $R^4$-($C_1$-$C_6$)-Alkoxy-CO oder einen Rest der allgemeinen Formel III

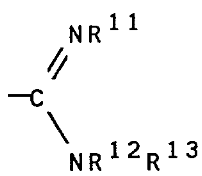 (III)

stehen;

$R^6$ für ($C_1$-$C_{10}$)-Alkyl, ($C_2$-$C_{10}$)-Alkenyl, $R^4$ oder $R^4$-($C_1$-$C_6$)-Alkyl steht;

$R^7$ und $R^8$ unabhängig voneinander die für $R^5$ angegebenen Bedeutungen haben oder für ($C_1$-$C_6$)-Alkylsulfonyl oder $R^4$-S(O)$_2$ stehen oder $R^7$ und $R^8$ zusammen mit dem Stickstoffatom, an das sie gebunden sind, einen Rest der allgemeinen Formel IV

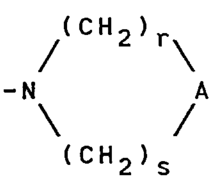 (IV)

bilden, der an den $CH_2$-Gruppen durch einen oder mehrere gleiche oder verschiedene Reste $R^{10}$ substituiert sein kann und worin r und s unabhängig voneinander für die Zahlen 1, 2, 3 oder 4 stehen, aber die Summe r + s kleiner als 6 ist;

$R^{7'}$ und $R^{8'}$ unabhängig von $R^7$ und $R^8$ die für $R^7$ und $R^8$ angegebenen Bedeutungen haben;

$R^9$ eine der Bedeutungen von $R^5$ hat, aber nicht für den Rest der allgemeinen Formel III steht;
$R^{10}$ für $(C_1-C_6)$-Alkyl, $(C_3-C_7)$-Cycloalkyl, den Rest eines 5- bis 7-gliedrigen Heterocyclus mit einem bis drei gleichen oder verschiedenen Heteroatomen aus der Reihe Stickstoff, Sauerstoff und Schwefel, der aromatisch, teilweise gesättigt oder vollständig gesättigt sein kann, $R^4$, Halogen, $OR^5$, $SR^{5'}$, $S(O)_p-R^{6'}$ wobei p für 1 oder 2 steht, $NR^7R^8$, die Hydroximinogruppe, die Oxogruppe, Cyan, Nitro, $COOR^9$, $CONR^{7'}R^{8'}$ oder $P(O)(OH)_2$ steht;
die Reste $R^{11}$, $R^{12}$ und $R^{13}$ unabhängig voneinander für Wasserstoff, $(C_1-C_6)$-Alkyl, $(C_3-C_7)$-Cycloalkyl, $R^4$, $(C_1-C_6)$-Alkanoyl, $(C_1-C_6)$-Alkyl-$S(O)_2$ oder $R^4$-$S(O)_2$ stehen;
$R^{14}$ für $(C_1-C_4)$-Alkyl, Benzyl, Phenyl, $(C_1-C_6)$-Alkanoyl, $(C_1-C_6)$-Alkyl-$S(O)_2$ oder $R^4$-$S(O)_2$ steht;
A für eine Methylengruppe, die durch einen oder zwei gleiche oder verschiedene Reste $R^{10}$ substituiert sein kann, für Sauerstoff, Schwefel oder $NR^{14}$ steht;
X für $CR^{20}R^{21}$ steht und $R^{20}$ und $R^{21}$ unabhängig von den Bedeutungen der Reste $R^1$ und $R^2$ die für $R^1$ und $R^2$ angegebenen Bedeutungen haben;
Y für $CR^{22}R^{23}$ steht und $R^{22}$ und $R^{23}$ unabhängig von den Bedeutungen der Reste $R^1$ und $R^2$ die für $R^1$ und $R^2$ angegebenen Bedeutungen haben;
Z für $CR^{24}R^{25}$ steht und $R^{24}$ und $R^{25}$ unabhängig von den Bedeutungen der Reste $R^1$ und $R^2$ die für $R^1$ und $R^2$ angegebenen Bedeutungen haben;
wobei auch Reste der Gruppen W, X, Y und Z über eine Bindung verknüpft sein können, so daß ein Bicyclus oder Tricyclus gebildet wird;
und ihrer tautomeren Formen sowie ihrer pharmakologisch verträglichen Salze als pharmakologische Wirkstoffe.

**2.** 2-Amino-1,3-thiazepin gemäß Anspruch 1, dadurch gekennzeichnet, daß W, X, Y und Z in der allgemeinen Formel I für die $CH_2$-Gruppe stehen.

**3.** Verwendung von 2-Amino-1,3-thiazepinen gemäß Anspruch 1 und/oder 2 zur Vorbeugung und Behandlung von Krankheiten, die durch einen erhöhten Stickstoffmonoxid-Spiegel bedingt sind.

**4.** Verwendung gemäß Anspruch 3 zur Behandlung und Vorbeugung pathologischer Blutdruckabfälle, insbesondere beim septischen Schock und der Krebstherapie mit Cytokinen.

**5.** Verwendung gemäß Anspruch 3 zur Behandlung und Vorbeugung von entzündlichen Erkrankungen, insbesondere Colitis ulcerosa.

**6.** Verwendung gemäß Anspruch 3 zur Behandlung und Vorbeugung von Infarktschäden und/oder Reperfusionsschäden.

**7.** Verwendung gemäß Anspruch 3 zur Behandlung und Vorbeugung von Transplantat-Abstoßungsreaktionen.

**8.** Verwendung gemäß Anspruch 3 zur Behandlung und Vorbeugung von Erkrankungen des Nervensystems aus der Reihe Morbus Alzheimer, Epilepsie und Migräne.

**9.** 2-Amino-1,3-thiazepine der allgemeinen Formel I,

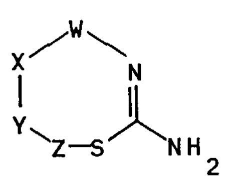

(I)

in der W für $CR^1R^2$ steht und
$R^1$ und $R^2$ unabhängig voneinander für Wasserstoff, Halogen, $R^3$, $R^4$, $OR^5$, $SR^{5'}$, $S(O)_p-R^6$, wobei p für 1 oder 2 steht, $NR^7R^8$, Cyan, Nitro, $COOR^9$, $CONR^{7'}R^{8'}$ oder $P(O)(OH)_2$ stehen; oder $R^1$ und $R^2$ zusammen für $(C_1-C_6)$-Alkyliden oder $(C_3-C_7)$-Cycloalkyliden, die auch jeweils einen oder mehrere Reste $R^{10}$ tragen können, oder $R^1$ und $R^2$ zusammen für =O, =S, =NH, $=NR^3$ oder =NOH stehen; oder $R^1$ und $R^2$ zusammen mit dem Kohlenstoffatom, an das sie gebunden sind, einen Rest der allgemeinen Formel II

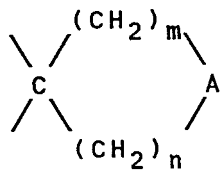

(II)

bilden, der vollständig oder teilweise dehydriert sein kann, der auch benzanelliert sein kann, der durch einen oder mehrere gleiche oder verschiedene Reste $R^{10}$ substituiert sein kann und worin m und n unabhängig voneinander für die Zahlen 0, 1, 2, 3, 4 oder 5 stehen, aber die Summe m + n einen der Werte 1, 2, 3, 4 oder 5 hat; oder $R^1$ oder $R^2$ eine freie Valenz bedeutet, die mit einer ebensolchen freien Valenz an einem Atom, das direkt an das $R^1$ und $R^2$ tragende Kohlenstoffatom gebunden ist, eine Doppelbindung bildet;

$R^3$ für ($C_1$-$C_{10}$)-Alkyl, ($C_2$-$C_{10}$)-Alkenyl, ($C_2$-$C_{10}$)-Alkinyl; ($C_3$-$C_7$)-Cycloalkyl, ($C_9$-$C_{11}$)-Benzocycloalkyl oder den Rest eines 4- bis 10-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, wobei der Alkyl-, Alkenyl-, Alkinyl-, Cycloalkyl- und der heterocyclische Rest durch einen oder mehrere gleiche oder verschiedene Reste $R^{10}$ substituiert sein können;

$R^4$ für Phenyl oder Naphthyl steht, die durch ein bis drei gleiche oder verschiedene Reste aus der Reihe ($C_1$-$C_4$)-Alkyl, ($C_1$-$C_4$)-Alkoxy, Halogen, Nitro, Trifluormethyl, Cyan, Hydroxy, ($C_1$-$C_4$)-Alkoxycarbonyl, Carboxyl, Carbamoyl, ($C_1$-$C_4$)-Alkyl-$S(O)_q$, wobei q für 0, 1 oder 2 steht, Amino, ($C_1$-$C_4$)-Alkylcarbonylamino, ($C_1$-$C_4$)-Alkylamino und Di(($C_1$-$C_4$)-Alkyl)amino substituiert sein können;

$R^5$ und $R^{5'}$ unabhängig voneinander für Wasserstoff, ($C_1$-$C_{10}$)-Alkyl, in dem die Alkylkette auch durch ein bis drei Sauerstoffatome unterbrochen sein kann, ($C_2$-$C_{10}$)-Alkenyl, ($C_3$-$C_7$)-Cycloalkyl, $R^4$, $R^4$-($C_1$-$C_6$)-Alkyl, den Rest eines 5- bis 7-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, ($C_1$-$C_{10}$)-Alkanoyl, $R^4$-CO, $R^4$-($C_1$-$C_6$)-Alkyl-CO, ($C_1$-$C_{10}$)-Alkyloxycarbonyl, $R^4$O-CO, $R^4$-($C_1$-$C_6$)-Alkoxy-CO oder einen Rest der allgemeinen Formel III

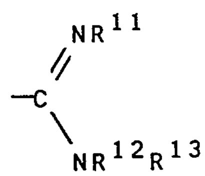

(III)

stehen;

$R^6$ für ($C_1$-$C_{10}$)-Alkyl, ($C_2$-$C_{10}$)-Alkenyl, $R^4$ oder $R^4$-($C_1$-$C_6$)-Alkyl steht;

$R^7$ und $R^8$ unabhängig voneinander die für $R^5$ angegebenen Bedeutungen haben oder für ($C_1$-$C_6$)-Alkylsulfonyl oder $R^4$-$S(O)_2$ stehen oder $R^7$ und $R^8$ zusammen mit dem Stickstoffatom, an das sie gebunden sind, einen Rest der allgemeinen Formel IV

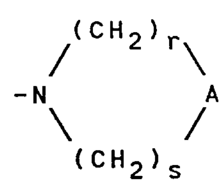

(IV)

bilden, der an den $CH_2$-Gruppen durch einen oder mehrere gleiche oder verschiedene Reste $R^{10}$ substituiert sein kann und worin r und s unabhängig voneinander für die Zahlen 1, 2, 3 oder 4 stehen, aber die Summe r + s kleiner als 6 ist;

$R^{7'}$ und $R^{8'}$ unabhängig von $R^7$ und $R^8$ die für $R^7$ und $R^8$ angegebenen Bedeutungen haben;

$R^9$ eine der Bedeutungen von $R^5$ hat, aber nicht für den Rest der allgemeinen Formel III steht;
$R^{10}$ für ($C_1$-$C_6$)-Alkyl, ($C_3$-$C_7$)-Cycloalkyl, den Rest eines 5- bis 7-gliedrigen Heterocyclus mit einem bis drei gleichen oder verschiedenen Heteroatomen aus der Reihe Stickstoff, Sauerstoff und Schwefel, der aromatisch, teilweise gesättigt oder vollständig gesättigt sein kann, $R^4$, Halogen, $OR^5$, $SR^{5'}$, $S(O)_p$-$R^6$, wobei p für 1 oder 2 steht, $NR^7R^8$, die Hydroximinogruppe, die Oxogruppe, Cyan, Nitro, $COOR^9$, $CONR^{7'}R^{8'}$ oder $P(O)(OH)_2$ steht;
die Reste $R^{11}$, $R^{12}$ und $R^{13}$ unabhängig voneinander für Wasserstoff, ($C_1$-$C_6$)-Alkyl, ($C_3$-$C_7$)-Cycloalkyl, $R^4$, ($C_1$-$C_6$)-Alkanoyl, ($C_1$-$C_6$)-Alkyl-$S(O)_2$ oder $R^4$-$S(O)_2$ stehen;
$R^{14}$ für ($C_1$-$C_4$)-Alkyl, Benzyl, Phenyl, ($C_1$-$C_6$)-Alkanoyl, ($C_1$-$C_6$)-Alkyl-$S(O)_2$ oder $R^4$-$S(O)_2$ steht;
A für eine Methylengruppe, die durch einen oder zwei gleiche oder verschiedene Reste $R^{10}$ substituiert sein kann, für Sauerstoff, Schwefel oder $NR^{14}$ steht;
X für $CR^{20}R^{21}$ steht und $R^{20}$ und $R^{21}$ unabhängig von den Bedeutungen der Reste $R^1$ und $R^2$ die für $R^1$ und $R^2$ angegebenen Bedeutungen haben;
Y für $CR^{22}R^{23}$ steht und $R^{22}$ und $R^{23}$ unabhängig von den Bedeutungen der Reste $R^1$ und $R^2$ die für $R^1$ und $R^2$ angegebenen Bedeutungen haben;
Z für $CR^{24}R^{25}$ steht und $R^{24}$ und $R^{25}$ unabhängig von den Bedeutungen der Reste $R^1$ und $R^2$ die für $R^1$ und $R^2$ angegebenen Bedeutungen haben;
wobei auch Reste der Gruppen W, X, Y und Z über eine Bindung verknüpft sein können, so daß ein Bicyclus oder Tricyclus gebildet wird;
und ihrer tautomeren Formen sowie ihrer pharmakologisch verträglichen Salze,
wobei aber,

a) wenn $R^{20}$ und $R^{21}$ für Wasserstoff stehen, nicht

a1) gleichzeitig $R^1$, $R^2$, $R^{22}$, $R^{23}$, $R^{24}$ und $R^{25}$ für Wasserstoff stehen können;
a2) gleichzeitig $R^{22}$, $R^{24}$ und $R^{25}$ für Wasserstoff, $R^{23}$ für die Carboxylgruppe und $R^1$ und $R^2$ zusammen für =O stehen können;
a3) gleichzeitig $R^{22}$, $R^{23}$ und $R^{24}$ für Wasserstoff, $R^{25}$ für Brommethyl und $R^1$ und $R^2$ zusammen für =O stehen können;
a4) gleichzeitig $R^1$, $R^2$, $R^{24}$ und $R^{25}$ für Wasserstoff und $R^{22}$ und $R^{23}$ zusammen für =O stehen können;
a5) gleichzeitig $R^1$ für Wasserstoff und $R^2$ für Butyl stehen und $R^{22}$, $R^{23}$, $R^{24}$ und $R^{25}$ zusammen mit den beiden Kohlenstoffatomen, an die sie gebunden sind, einen 2-Oxo-1,2-dihydrochinolin-3,4-diyl-Rest bilden können;

b) wenn $R^{20}$, $R^{21}$, $R^{22}$ und $R^{23}$ zusammen mit den beiden Kohlenstoffatomen, an die sie gebunden sind, einen 1,2-Phenylen-Rest oder einen 4-Chlor-1,2-phenylen-Rest bilden, nicht

b1) gleichzeitig $R^1$, $R^2$ und $R^{24}$ für Wasserstoff und $R^{25}$ für Phenyl stehen können;
b2) gleichzeitig $R^1$, $R^{24}$ und $R^{25}$ für Wasserstoff und $R^2$ für Phenyl stehen können;
b3) gleichzeitig $R^1$ und $R^2$ zusammen für =O und $R^{24}$ und $R^{25}$ zusammen für =O stehen können.

**10.** Pharmazeutisches Präparat, dadurch gekennzeichnet, daß es ein oder mehrere der 2-Amino-1,3-thiazepine der allgemeinen Formel I gemäß Anspruch 1 und/oder 2 und/oder ein oder mehrere pharmakologisch annehmbare Salze davon als Wirkstoff zusammen mit pharmazeutisch annehmbaren Träger- und Zusatzstoffen und gegebenenfalls noch einen oder mehrere andere pharmakologische Wirkstoffe enthält.

Europäisches Patentamt

# EUROPÄISCHER RECHERCHENBERICHT

Nummer der Anmeldung
EP 95 11 8404

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,X | PHARMAZIE,<br>Bd. 48, 1993, BERLIN DE,<br>Seiten 812-816, XP000567766<br>J. LESSEL:<br>* Beispiel 20C *<br>--- | 1 | C07D281/02<br>C07D283/02<br>C07D285/36<br>A61K31/55 |
| X | CHEMICAL ABSTRACTS, vol. 114, no. 11, 18.März 1991<br>Columbus, Ohio, US;<br>abstract no. 100775x,<br>XP002001877<br>* STN Database (CA): RN: 132397-26-3 * | 1 | |
| D | & METALLOORG. KHIM.,<br>Bd. 3 , Nr. 5, 1990,<br>Seiten 1115-1126,<br>L.I. KIRKOVSKII:<br>--- | | |
| X | CHEMICAL ABSTRACTS, vol. 94, no. 19, 11.Mai 1981<br>Columbus, Ohio, US;<br>abstract no. 156850d,<br>XP002001878<br>* STN Database (CA): RN: 77250-74-9 * | 1 | RECHERCHIERTE SACHGEBIETE (Int.Cl.6) |
| D | & HETEROCYCLES,<br>Bd. 15, Nr. 1, 1981,<br>Seiten 399-402,<br>T. KATO ET AL.:<br>---<br>-/-- | | C07D<br>A61K |

Der vorliegende Recherchenbericht wurde für alle Patentansprüche erstellt

| Recherchenort | Abschlußdatum der Recherche | Prüfer |
|---|---|---|
| BERLIN | 30.April 1996 | Frelon, D |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 95 11 8404

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X<br><br><br><br><br><br><br>D | CHEMICAL ABSTRACTS, vol. 88, no. 17, 24.April 1978<br>Columbus, Ohio, US;<br>abstract no. 121131u,<br>XP002001879<br>* STN Database (CA): RN: 65765-80-2; 65765-51-7; 65765-48-2 *<br>& HELVETICA CHIMICA ACTA,<br>Bd. 60, Nr. 8, 1977, BASEL CH,<br>Seiten 2872-2880,<br>J.B. BREAM ET AL.:<br>--- | 1 | |
| X,D | DATABASE WPI<br>Week 7124<br>Derwent Publications Ltd., London, GB;<br>AN 71-41795s<br>XP002001882<br>& JP-B-46 021 713 (TATSUKICHI KOBAYASHI) , 19.Juni 1971<br>* STN Database (CA): RN: 34488-27-2 *<br>--- | 1 | |
| X<br><br><br><br><br><br>D | CHEMICAL ABSTRACTS, vol. 71, no. 11, 15.September 1969<br>Columbus, Ohio, US;<br>abstract no. 49916h,<br>XP002001880<br>* 4. Beispiel *<br>& UKR. KHIM. ZH.,<br>Bd. 35, Nr. 5, 1969,<br>Seiten 526-529,<br>Y.V. MIGALINA ET AL.:<br>---<br>-/-- | 1 | RECHERCHIERTE SACHGEBIETE (Int.Cl.6) |

Der vorliegende Recherchenbericht wurde für alle Patentansprüche erstellt

| Recherchenort | Abschlußdatum der Recherche | Prüfer |
|---|---|---|
| BERLIN | 30.April 1996 | Frelon, D |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 95 11 8404

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X<br><br><br><br><br><br>D | CHEMICAL ABSTRACTS, vol. 70, no. 11, 17.März 1969<br>Columbus, Ohio, US;<br>abstract no. 47380j,<br>XP002001881<br>* STN Database (CA): RN: 21463-30-9 *<br>& ARCHIV DER PHARMAZIE,<br>Bd. 301, Nr. 10, 1968, WEINHEIM DE,<br>Seiten 750-762,<br>H.W. SCHUBERT ET AL.: | 1 | |
| X,D | DE-A-19 65 309 (SHIONOGI & CO. LTD.)<br>* STN Database (CA): RN: 28664-71-3 * | 1 | |
| X | DE-A-26 21 907 (SANDOZ-PATENT-GMBH)<br>* Beispiele 30,31 * | 1 | |
| A,D | WO-A-94 12165 (THE WELLCOME FOUNDATION LIMITED)<br>* das ganze Dokument * | 1,3,10 | |
| | | | RECHERCHIERTE SACHGEBIETE (Int.Cl.6) |
| A<br><br>D | EP-A-0 558 468 (WASHINGTON UNIVERSITY)<br>* Zusammenfassung *<br>& US-A-5 246 971 | 1,3,10 | |
| A | EP-A-0 261 439 (MECT CORPORATION)<br>* Ansprüche * | 1,3,10 | |
| A | FR-A-2 271 818 (MORTON-NORWICH PRODUCTS, INC.)<br>* Beispiel 11 * | 1 | |

Der vorliegende Recherchenbericht wurde für alle Patentansprüche erstellt

| Recherchenort | Abschlußdatum der Recherche | Prüfer |
|---|---|---|
| BERLIN | 30.April 1996 | Frelon, D |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P04C03)